# EUROPEAN PATENT APPLICATION

(11) **EP 1 609 482 A1**
(43) Date of publication of application: **28.12.2005**
(21) Application number: 04721332.7
(22) Date of filing: 17.03.2004
(51) Int. Cl.: A61K 48/00, A61K 31/7088, A61K 47/42, A61K 9/51, A61K 38/36, A61K 38/37, A61P 7/04

(54) **DRUG FOR TREATING HEMOPHILIA AND METHOD OF TREATING HEMOPHILIA USING THE SAME**

(30) Priority: 17.03.2003 JP 2003071788
(71) Applicant: Beacle Inc., Okayama-shi, Okayama 701-1221 (JP); VIB (Vlaams Interuniversitair Instituut voor Biotechnologie) VZW, 120 Zwijnaarde B9052 (BE); D. Collen Research Foundation vzw, 3000 Leuven (BE)
(72) Inventor: UEDA, Masakazu, Tokyo 1620837 (JP); KURODA, Shunichi, Suita-shi, Osaka 5650872 (JP); TANIZAWA, Katsuyuki, Toyono-gun, Osaka 5630214 (JP); SENOO, Masaharu, Okayama-shi, Okayama 7038273 (JP); KONDO, Akihiko, Kobe-shi, Hyogo 6570015 (JP); VANDENDRIESSCHE, Thierry, Korbeek-Lo 3360 (BE); CHUAH, Marinee, Korbeek-Lo 3360 (BE)
(74) Representative: Jones, Helen M.M.
(86) International application number: PCT/JP2004/003560
(87) International publication number: WO 2004/082720

(57) **Abstract**

A therapeutic product or drug for therapy of hemophilia may be produced by a simplified method including embedding genes of the blood clotting factors VIII (IX) for therapy of hemophilia in hollow nano particles obtained on expressing the protein having a particle forming function, such as hepatitis B virus surface antigen protein, in eucaryotic cells. The drug so produced is able to introduce the genes of the blood clotting factors efficaciously into liver cells with the least risk of side effects.

## Description

### Using the Same

### Technical Field

This invention relates to a therapeutic product (drug) for treating hemophilia using hollow nano particles, and a method of treating hemophilia using this drug. More particularly, it relates to a therapeutic product (drug) comprising a substance enclosed in particles for transfer into cells, which substance may be specifically introduced into the cells for hemophilia treatment, and to a method of treating hemophilia using the product.

This application claims priority of Japanese Patent Application 2003-071788, filed in Japan on March 17, 2003, which is incorporated by reference herein.

### Background Art

In the field of medicine in recent years, development of a drug, directly acting on the affected site to display a high therapeutic efficacy with lesser side effects, is proceeding briskly. In particular, a method termed the drug delivery system (DDS) is attracting notice as being a method for specifically transporting effective components, such as those of a drug, to a target cell or tissue for causing the components to act on the target site.

On the other hand, in the field of molecular cell biology of recent years, investigations into the transfer of genes to specified cells are also going on as an indispensable technique. Moreover, with the progress of the human genome project, the hereditary background of various diseases has become demystified. Thus, if a method for gene transfer exhibiting high specificity relative to these cells or tissues is established at the present time, application of the method to the field of gene therapy would become possible.

Among the methods for introducing genes into the cells, there are known a method for turning a gene into a giant molecule which is then taken into the cells by endocytosis (a calcium phosphate method or a lipofectamine method) and a method for applying electrical pulse stimuli to the cell membrane to render it permeable to allow the genes to be taken into the cells (an electroporation method or a gene gun method). Both of these methods are currently practiced in experiments in the field of molecular biology.

These methods, while being simple, tend to injure the cells directly physically. Moreover, the site of gene transfer has to be exposed by surgical measures. For these reasons, the methods cannot be applied readily to the cells or tissues of living bodies. In addition, it is difficult to achieve the rate of transfer close to 100 % .

There is also known a liposome method as a method for introducing a substance with higher safety. This method may be applied to cells or tissues of a living body in that it does not injure the cells. However, the method suffers from a problem that it is difficult to confer high specificity for cells or tissues on liposome, which is a simple lipid, and that the rate of in-vivo gene transfer is markedly lower than a desired value.

There has recently been developed a technique of incorporating a gene of interest into a viral DNA to generate an infectious virus in order to effect gene introduction. This method is stirring up notice as an epoch-making method for gene therapy against a variety of hereditary and acquired diseases, in that the method does not expose the site of transfer to outside, may be applied to an individual and has transfer efficiency close to 100%.

For example, hemophilia is a hereditary disease having hemorrhage due to the deficiency of blood clotting factors as a main symptom. It is noted that hemophilia A is caused by the deficiency of the blood clotting factor VIII (anti-hemophilia factor), and that hemophilia B is caused by the deficiency of the blood clotting factor IX (Christmas factor). The hemophilia A and the hemophilia B are retained to be caused by gene disorder of the factors VIII and IX on the X-chromosomes, respectively. In general, the supplementary therapy by intravenous injection of the VIII (IX) factor drug is applied to a patient of hemophilia. In this connection, for constant expression of an amount of the clotting factors VIII (IX) close to the physiological level, there is proposed in e.g. the Japanese Patent Publication Kohyo 2002-527493 a technique of preparing an adeno-associated vector, inclusive of a sequence coding the factor VIII, and administering the so prepared vector to the patient of hemophilia A.

However, the gene transfer, employing the viral DNA, suffers a serious problem that the virus non-specifically infects a wide range of cells, so that genes are introduced into other than the target cells. There is also a possibility that the virus genome per se is incorporated into the chromosome to give rise to unforeseen side effects in future. Moreover, since the virus lacks cell or tissue specificity, it is necessary to administer the vector to e.g. the portal vein, or to interconnect the sequence coding the factor VIII and a control sequence specifically transcribed depending on the histological pattern, if the factor VIII is to be expressed in the liver.

The present inventors have also proposed in the Japanese Laid-Open Patent Publication 2001-316298 a method of specifically safely transporting and introducing a substance, such as genes, protein or compounds, to a target cell or tissue, using hollow nano particles of a protein, exhibiting the capability of forming particles, and into which have been introduced bio-recognition molecules.

With the technique disclosed in this Japanese Laid-Open Patent Publication 2001-316298, a variety of substances may be transported with the aid of hollow nano particles. It is now incumbent, as a further task, to develop a drug for therapy of specified diseases, such as hemophilia, using this technique.

### Disclosure of the Invention

In view of the above-depicted status of the art, it is an object of the present invention to provide a drug for treating hemophilia, in which genes of blood clotting factors may efficaciously be introduced into liver cells by a simple introducing method, with the least risk of side effects, and a method of treating hemophilia using the drug.

The present inventors have conducted perseverant researches and, through experiments of intravenously injecting hepatitis B virus surface antigen particles, containing the genes of blood clotting factors VIII and IX, to a test animal implanted with human liver cancer cells, have found that the genes can be specifically introduced into tissue parts derived from the human liver to express the blood clotting factors to give rise to favorable results in treating the hemophilia. This finding has led to completion of the present invention.

According to the present invention, there is provided a drug for therapy of hemophilia comprising hollow nano particles formed of protein exhibiting a particle forming capability, and genes for therapy of hemophilia embedded in the hollow nano particles.

According to the present invention, there is also provided a drug for therapy of hemophilia comprising hollow nano particles formed by introducing bio-recognition molecules into protein particles obtained on expressing the protein in eucaryotic cells, and genes for therapy of hemophilia embedded in the hollow nano particles.

The protein, forming the particles, may be exemplified by a hepatitis B virus surface antigen protein. This protein, when expressed in the eucaryotic cells, is expressed and accumulated as membrane protein on a vesicle membrane and released as particles. The so produced hollow nano particles are able to recognize the liver cells and to transport a substance in the particles specifically to the liver cells, so that, by embedding genes for therapy of hemophilia, specifically, the blood clotting factors VIII or IX, in the hollow particles, these genes may be specifically expressed in the liver cells.

The drug for therapy of the present invention may effectively treat the hemophilia by a simpler method of intravenous injection and may directly be put to clinical use with the least risk of side effects.

The method for treating the hemophilia according to the present invention treats the hemophilia by administering the drug for therapy of hemophilia according to the present invention.

Other objects and advantages of the present invention will become more apparent from the following explanation of preferred embodiments thereof especially when read in conjunction with the accompanying drawings.

### Brief Description of the Drawings

Fig.1 schematically shows different protein areas of an HBsAg gene according to an embodiment of the present invention.
Fig.2 schematically shows the operation of expression and purification of HBsAg particles employing recombinant yeast according to an embodiment of the present invention.
Fig.3 shows the effect of expression of the blood clotting factor VIII by HBsAg particles containing hFVIII genes according to an embodiment of the present invention.
Fig.4 shows the effect of expression of the blood clotting factor IX by HBsAg particles containing hFIX genes according to an embodiment of the present invention.

### Best Mode for Carrying out the Invention

The hollow nano particles, embodying the present invention, introduce bio-recognition molecules into the protein having the particle forming capability to render it possible to specifically transport genes coding the clotting factors VIII and IX to desired cells or tissues, for example, to liver cells or liver tissues. The protein having the particle forming capability may, for example, be sub-virus particles, obtained from a variety of viruses. The protein may be exemplified by hepatitis B virus (HBV) surface antigen protein.

The protein particles, formed of protein having such particle forming capability, may be exemplified by those obtained by expressing the protein in an eucaryotic cell. In short, if the protein having the particle forming capability is expressed in the eucaryotic cell, the protein is expressed and accumulated as membrane protein on the vesicle membrane so as to be released as particles. The eucaryotic cell may be exemplified by yeast, recombinant yeast, insect cells and animal cells.

As shown in Examples, described subsequently, the present inventors have found and reported that, by expressing the aforementioned HBV surface antigen L protein in the recombinant yeast, a large number of substantially elliptically-shaped hollow particles, each being of a short diameter of approximately 20 nm and a long diameter of approximately 150 nm, and each having the HBV surface antigen L protein buried in a dual lipid film of yeast origin, may be formed (J. Bio. Chem., Vol.267, No.3, 1953-1961, 1992). Since these particles contain neither HBV genomes nor HBV protein and hence do not act as viruses, the particles are highly safe to human bodies. Moreover, these particles display receptors specific for liver cells, which receptors exhibit the extremely high infective power to the liver cells of HBV, on the surfaces thereof, and hence the particles exhibit high efficacy as a transporter for specific transportation of a substance to the liver cells.

The method for forming protein particles using the recombinant yeast is convenient in that particles may be produced with high efficacy from the soluble protein in the cell lysate.

On the other hand, the method employing insect cells and animal cells may be said to be a desirable method for mass-producing foreign protein because these cells are eucaryotic cells closer to the cells of higher animals than to the yeast cell, and also because the insect cells and animal cells are able to reproduce a high-order structure, such as sugar chain, that cannot be reproduced with the yeast. The conventional system of insect cells employs the baculo virus, and is accompanied by expression of viruses, such that cells are dead or dissolved at the time of protein expression. Hence, there arises a problem that protein expression is carried out in succession, or that the protein is decomposed by protease isolated from the dead cells. Moreover, if protein is expressed on secretion, the bovine fetal serum, contained in the culture medium, is mixed in large quantities to render it difficult to purify the particles. However, an insect cell system, not employing the baculo viruses, and which permits serum-free culturing, has recently been developed by Invitrogen Inc. and is being put for sale. Thus, with the use of this insect cell system, it is possible to realize protein particles which may be purified extremely readily and which allow the reproduction of a high-order structure.

With the hollow nano particles of the present invention, it is possible to transport and introduce a substance to optional cells or tissues, other than the liver cells, with extremely high specificity, by modifying the receptors on the particle surfaces, obtained by the above-described various methods, into optional bio-recognition molecules.

Of course, the protein exhibiting the particle forming capability is not limited to the aforementioned hepatitis B virus surface antigen protein, and may be any natural proteins derived from animal cells, plant cells, viruses or bacteria, or any of a variety of synthetic proteins. In case e.g. an antigen protein, derived from viruses, is likely to induce an antibody in a living body, such protein, modified to diminish its antigenicity, may be used as bio-recognition molecules.

The bio-recognition molecules, introduced into the proteins, exhibiting the particle forming capability, may preferably be enumerated by, for example, growth factors, cell function adjustment molecules, such as cytokine, cell surface antigens, tissue specific antigens, molecules for recognizing cells or tissues, such as receptors, molecules derived from viruses and microorganisms, antibodies, sugar chains or lipids. These may be suitably selected and used in dependence upon the target cells or tissues.

According to the present invention, the genes coding blood clotting factors VIII and IX, desired to be introduced into optional cells or tissues, in this case, liver cells or tissues, are enclosed in the above-described hollow nano particles to give a substance transporter for therapy of hemophilia.

For introducing the gene into the aforementioned hollow nano particles, any of a variety of methods used in routine techniques adopted in chemical experiments or in experiments in molecular biology, may be used. Examples of these methods include an electroporation method, an ultrasonic method, a simple diffusion method, or a method employing charged lipids.

Using these hollow nano particles, or the substance transporter, specific substance transfer to the cells or tissues in vivo or in vitro becomes possible. In addition, it becomes possible to introduce a substance to specified cells or tissues, with the aid of the hollow nano particles, or the substance transporter, as a method for treating various diseases, or as one step thereof.

The efficacy of the drug of the present invention has been actually confirmed by animal experiments, as indicated by Examples which will now be explained. In these Examples, the efficacy of the drug of the present invention, containing genes coding the blood clotting factors VIII and IX, was confirmed by first administering the drug to a nude mouse, transplanted with a cell derived from the human liver cancer, and by then measuring the expression level of the blood clotting factors VIII and IX in the serum. Although the drug was administered intravenously, the drug may also be administered orally, intramuscularly, intraperitoneally or subcutaneously.

The present invention will now be explained in detail with reference to specified Examples as reference is made to the drawings. The present invention is not limited to the following Examples, and may encompass various changes, substitutions or equivalents thereof without departing from the purport and the scope of the invention as defined in the claims.

### Examples

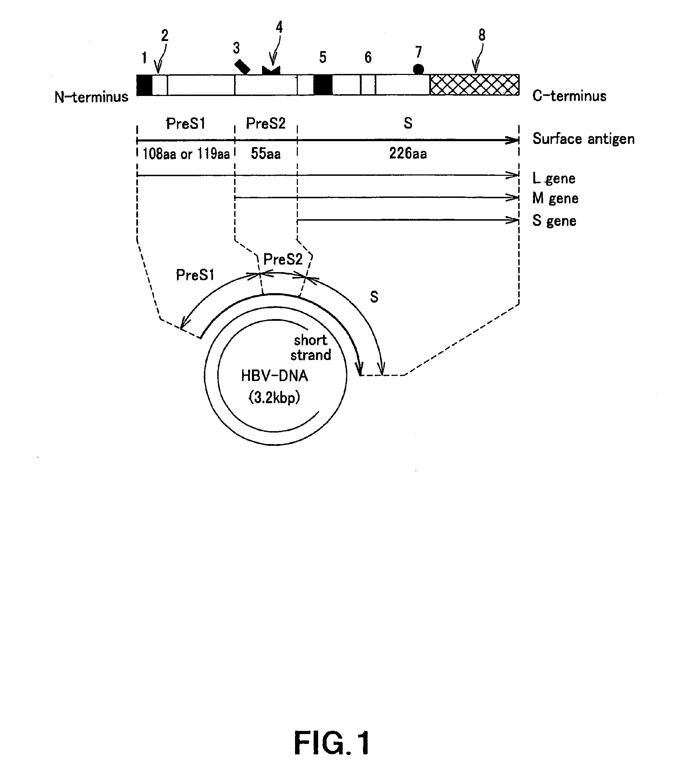

In the Examples, that follow, HBsAg denotes a hepatitis B virus surface antigen. Specifically, HBsAg is a coat protein of HBV. Referring to the schematic view of Fig. 1, there are three proteins in HBsAg, namely S-protein, M-protein and L-protein. Of these, the S-protein is a crucial coat protein common to the three proteins. The M-protein is a pre-S2 peptide, composed of 55 amino acids, and which is attached to the N-terminal side of the S-protein. The L-protein is a pre-S1 peptide, composed of 108 amino acids or 119 amino acids, and which is attached to the N-terminal side of the M-protein. The base sequence and the amino acid sequence of this L-protein are indicated by sequence numbers 1 and 2, respectively.

In a known manner, the pre-S1 domain of the L-protein of the HBsAg has a site for direct coupling to the liver cell, and plays a crucial role when the HBV is attached to the liver cell (Cell. Vol.46, 429-436, 1986: J. of Virol., Vol.73, 2052-2057, 1999).

When the protein HBsAg is expressed in the eucaryotic cell, the protein is expressed and accumulated as membrane protein on the vesicular membrane. The molecules of L-protein of HBsAg are flocculated together and take in the vesicular membrane in the course of the flocculation. The so flocculated molecules of L-protein of HBsAg are released as a particle to the lumen side in a budding fashion.

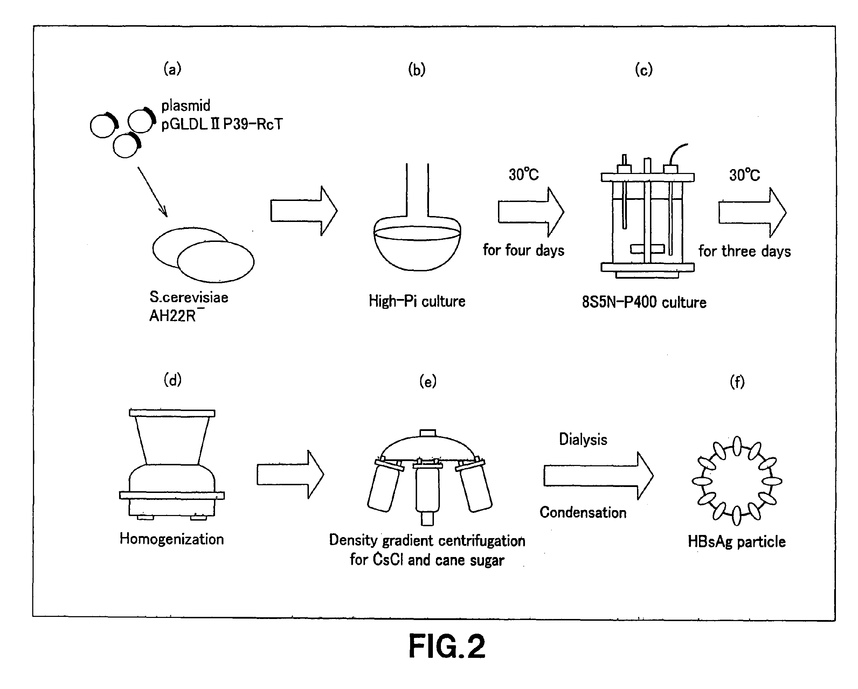

In the Examples that follow, the L-protein of HBsAg is used. Fig.2 schematizes the expression and the operations for purification of HBsAg particles described in the following Examples.

### Example 1

### Expression of HBsAg particles by recombinant yeast

Based on a literature entitled 'J. Bio. Chem., Vol.267, No.3, 1953-1961, 1992', reported by the present inventors, a recombinant yeast, holding L-protein expressing plasmid pGLDLIIP39-RcT (Saccharomyces Cerevisiae AH22R⁻ strain), was cultured in synthetic media High-Pi and 8S5N-P400, to express L-protein particles (Figs.2a and 2b). From the recombinant yeast, in the stationary growth phase (after approximately 72 hours), a whole cell extract was prepared, using a yeast protein extraction reagent, manufactured by Pierce Chemicals Co. Ltd. The proteins in the whole cell extract were separated from one another by sodium dodecyl sulfate-polyacrylamide gel electrophoresis (SDS-PAGE), and HBsAg in the sample was identified by silver staining. In this manner, HBsAg was verified to be a protein with a molecular weight of approximately 52 kDa.

### Example 2

### Purification of HBsAg particles from recombinant yeast

(1) The recombinant yeast (wet weight: 26g), cultured on a synthetic culture medium 8S5N-P400, was suspended in 100 ml of a buffer solution A (7.5M urea, 0.1M sodium phosphate (pH 7.2), 15 mM EDTA, 2 mM PMSF and 0.1 % Tween 80), and the yeast was homogenized with glass beads, using a bead beater (BEAD-BEATER). After homogenization, the supernatant was recovered by centrifugation (Figs.2c and 2d).
(2) The supernatant was then mixed with a 0.75-fold volume of 33% (w/w) PEG6000 and the resulting mixture was cooled with ice for 30 minutes. Then, pellets were recovered after centrifugation at 7000 rpm for 30 minutes. The pellets were then re-suspended in a buffer A solution not containing Tween 80.
(3) The solution following re-suspension was layered on CsCl exhibiting a density gradient in a range from 10 to 40%. The solution was then subjected to ultra-centrifugation at 28000 rpm for 16 hours. The centrifuged sample was separated into 12 fractions and the fraction containing HBsAg was identified by the Western blotting method, in which the first antibody was an anti-HBsAg monoclonal antibody. Further, the fraction containing HBsAg was dialyzed, using the buffer A solution not containing Tween 80.
(4) The solution (12 ml) obtained on dialysis in (3) was layered on sugar, exhibiting a density gradient in a range from 5 to 50%. The resultant mass was subjected to ultra-centrifugation at 28000 rpm for 16 hours. After centrifugation, the fraction containing the HBsAg was identified, as in (3). This fraction containing the HBsAg was dialyzed with a buffer A solution not containing urea nor Tween 80 and containing 0.85% NaCl in their stead ((2) to (4) in Fig.2e).
(5) The operation similar to that of (4) above was repeated. The as-dialyzed sample was condensed, using an ultra-filter Q2000, manufactured by Advantec Inc., and was stored in a refrigerator at 4ºC until use (Fig.2f). The results of the Western blotting (3) following CsCl equilibrium centrifugation indicated that HBsAg was a protein of the molecular weight of 52 kDa, exhibiting S-antigenicity. Ultimately, about 24 mg of purified HBsAg particles could be obtained from the cell lyzate of a wet weight of 26g, derived from the culture medium 2.5L.

The fractions from the sequence of the purifying operations were analyzed using silver staining SDS-PAGE. Additionally, for confirming that the protease of yeast origin was removed by the purification process, the HBsAg particles, obtained by (5), were incubated at 37ºC for 12 hours, and subjected to SDS-PAGE for 12 hours, followed by SDS-PAGE for identification by silver staining. As a result, it was confirmed that the protease derived from yeast was completely removed in the sequence of the purification steps.

### Example 3

### Enclosing hFVIII and HFIX genes into HBsAg particles (preparation of HBsAg particles including the hFVIII and hFIX genes embedded therein)

Into the HBsAg particles, prepared by the above method, genes (hFVIII and hFIX), coding the human blood clotting factors VIII (IX), as genes for therapy of hemophilia, were enclosed to produce HBsAg particles having embedded the genes (hFVIII and hFIX) as the drug according to the present invention.

In the present Example, pRRLsin.cPPT.CMV.FVIII.Wpre and pRRLsin.cPPT.AIb.FIX.Wpre (Human Gene Therapy, Vo1.13, 243-260, 2002), donated by Dr. L. Naldini of Torino University, were used as expression vectors for enclosing the hFVIII and hFIX genes in the HBsAg particles.

The HBsAg particles, having the hFVIII (hFIX) genes embedded therein, were prepared by introducing the above expression vectors into the HBsAg particles by the electroporation method. Specifically, 20 µg of the above expression vectors was added to 100 µg of the L-protein particles in the HBsAg particles dissolved in 500 µl of PBS (pH 7.2). The electroporation was carried out using a cuvette of 4 mm at 50V and 750 µF on a Gene Pulser II electroporation system (manufactured by Bio-Rad Co. Ltd.).

### Example 4

### Effect of expression of clotting factors VIII (IX) by HBsAg particles with embedded hFVIII (hFIX) genes in nude mice transplanted with human liver cancer

The effect of expression of the clotting factors VIII (IX) by the HBsAg particles, enclosing the hFVIII (hFIX) genes, prepared by the above Example, was verified on test animals.

In the present Example, 1 x 10⁷ cells, derived from human liver cancer Nue, were administered to both lateral dorsal hypodermal regions of a nude mouse (Balb/cnu/nu, female, five weeks old), purchased as test animal from Nippon Clair. Co. Ltd. The mouse was grown for approximately 5 to 6 weeks until a solid cancer grew to a size of about 1 cm diameter, to give a cancer-bearing mouse.

The HBsAg particles, having embedded therein about 20µg of the hFVIII (hFIX) gene expressing vectors, were then administered via a tail vein to the above cancer-bearing mouse, and changes with time of the quantity of the clotting factors VIII (IX) in the blood were measured by enzyme immunoassay (ELISA). The ELISA was carried out using an Asserachom VIIIC: Ag kit and an Asserachom IX: Ag kit, (manufactured by Diagnostica Stago Inc.), specific for the VIII and IX factors, respectively.

As negative control, a cancer-bearing mouse, obtained on administering 1×10⁷ cells derived from human colic cancer WiDr, was used, and the quantities of the clotting factors VIII (IX) in the plasma were measured in the same way as described above.

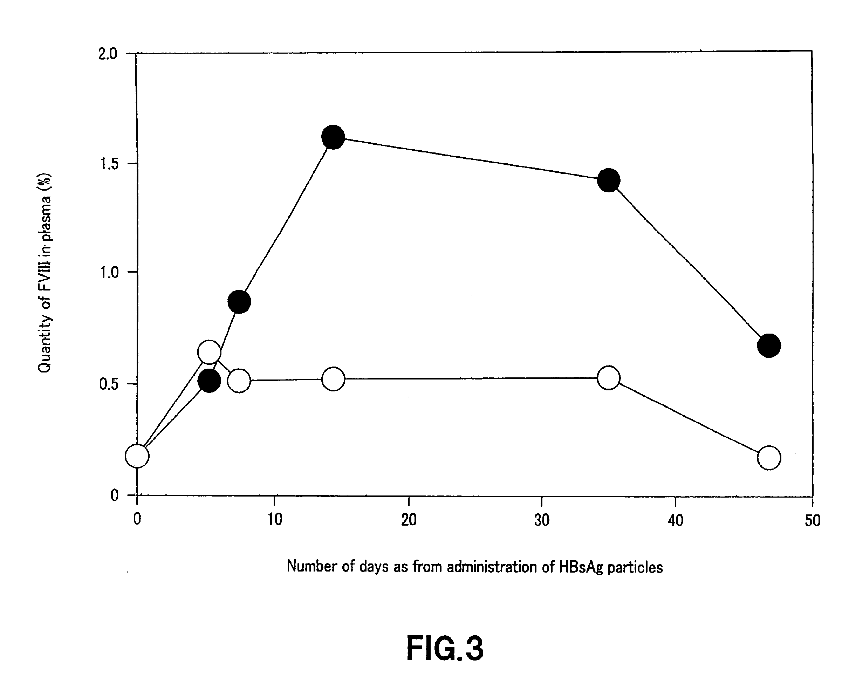

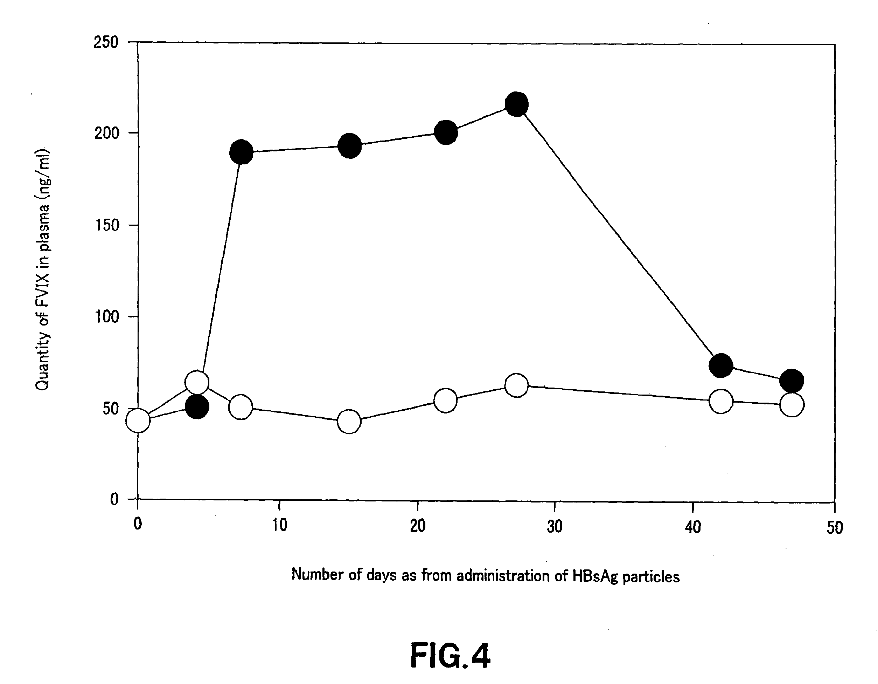

The transition of the proportion in % of the quantity of the clotting factor VIII in the plasma measured relative to the quantity of the clotting factor VIII in the positive control plasma of the above kit is shown in Fig.3. The concentration transition of the blood clotting factor IX in the plasma is shown in Fig.4. As may be seen from Figs.3 and 4, no changes with time were observed with the negative control, whereas, with the mouse, to which were administered the tumor cells (Nue), derived from the human liver cancer, expression of the clotting factors VIII and IX was observed after about ten days, and the level of the expression reached a value such that the state of the human patient is recovered from the 'severely ill' state to the 'median ill' state (Cur. Gene Therapy, Vol.1, 301 to 305, 2001). This level was then maintained at least for a month and subsequently lowered after about 40 days. This lowering in the expression is possibly attributable to the necrosis of the cancer due to tumorous cells (Nue).

Thus, it has been confirmed that the HBsAg particles, containing hFVIII (HFIX) genes, as the drug embodying the present invention, are able to introduce the genes into the human liver cells with high specificity and efficacy, and actually exhibit therapeutic efficacy against hemophilia. In addition, with the present experiments, the protocol for therapy of hemophilia by the HBsAg particles containing the FVIII (hFIX) genes could be established on the test animal level.

Although the pRRRLsinPPTCMVFVIIIpre (pRRLsinPPTAIbFIXpre) were used in the above Example as the vectors expressing the hFVIII (hfIX) genes, the present invention is not limited thereto, such that a variety of vectors described e.g. in the Publication 'Cur. Gene Therapy, Vol.1, 301-305, 2001' may also be used. For example, light and heavy chains of the clotting factor VIII may be incorporated into respective different vectors. In addition, the clotting factor VIII, lacking the B-domain, may also be incorporated in the vector for achieving comparable results.

### Industrial Applicability

The above-described drug for therapy of hemophilia according to the present invention is able to treat hemophilia efficaciously by a simple method of intravenous injection and may directly be put to clinical use with the least risk of side effects.

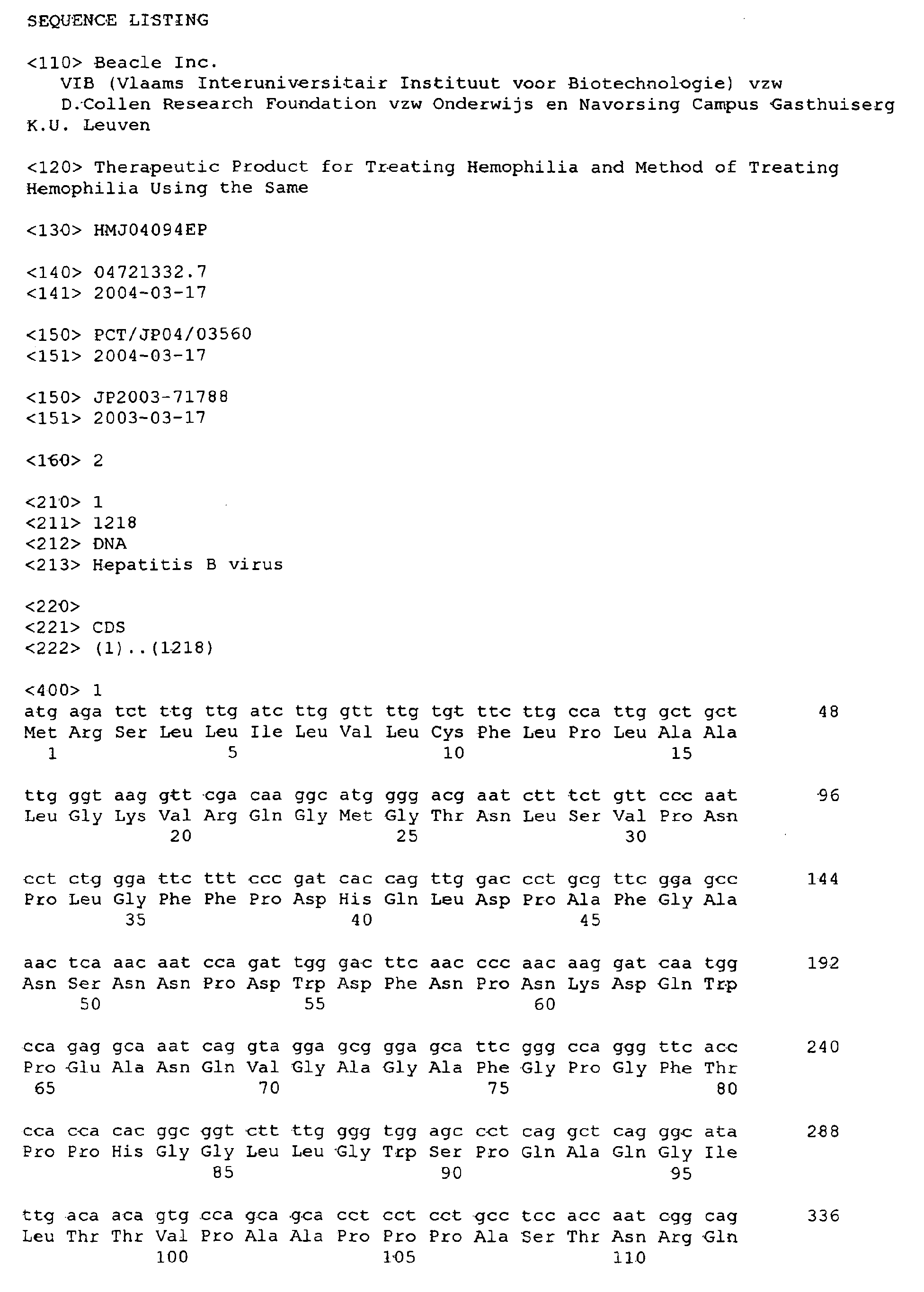

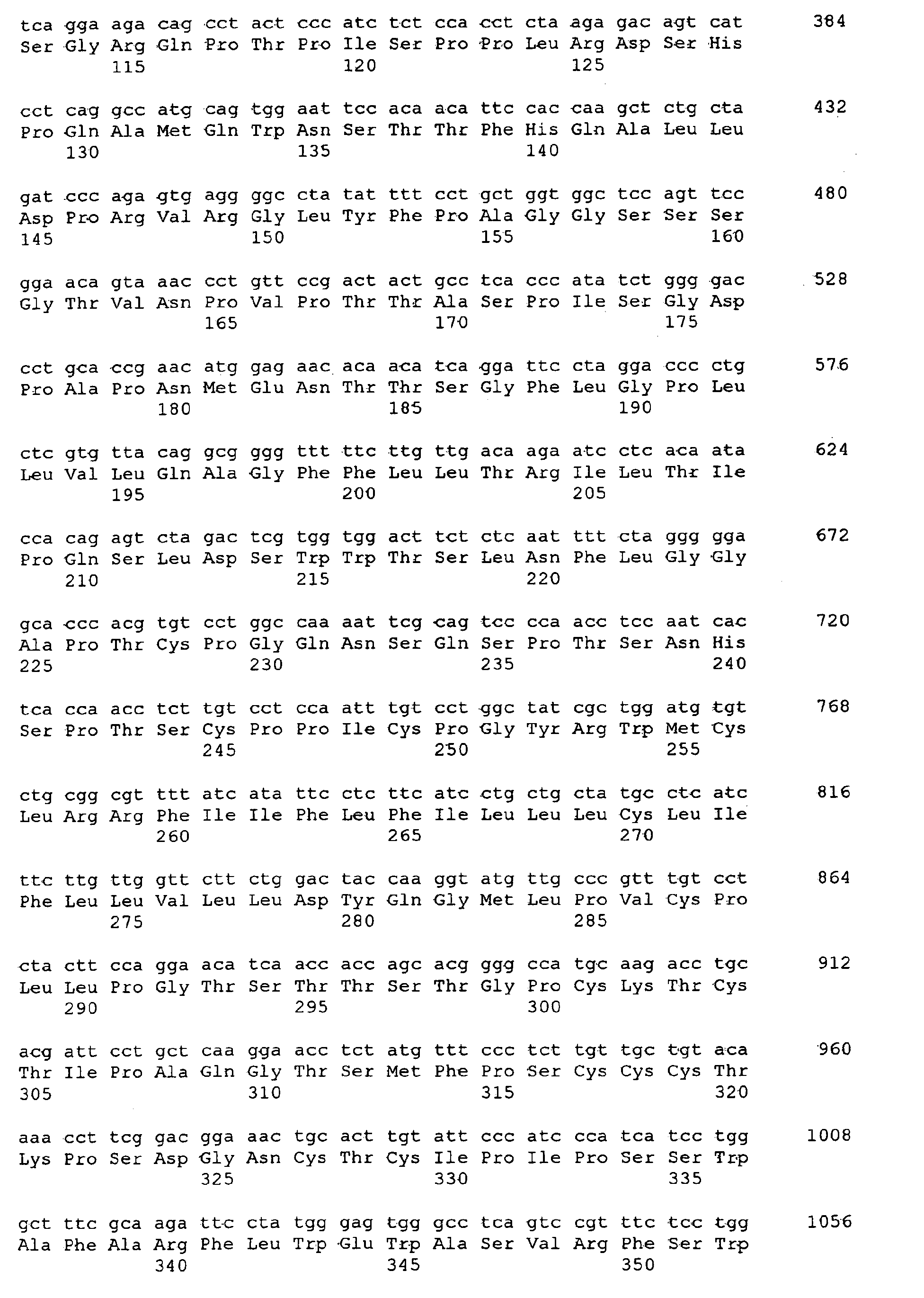

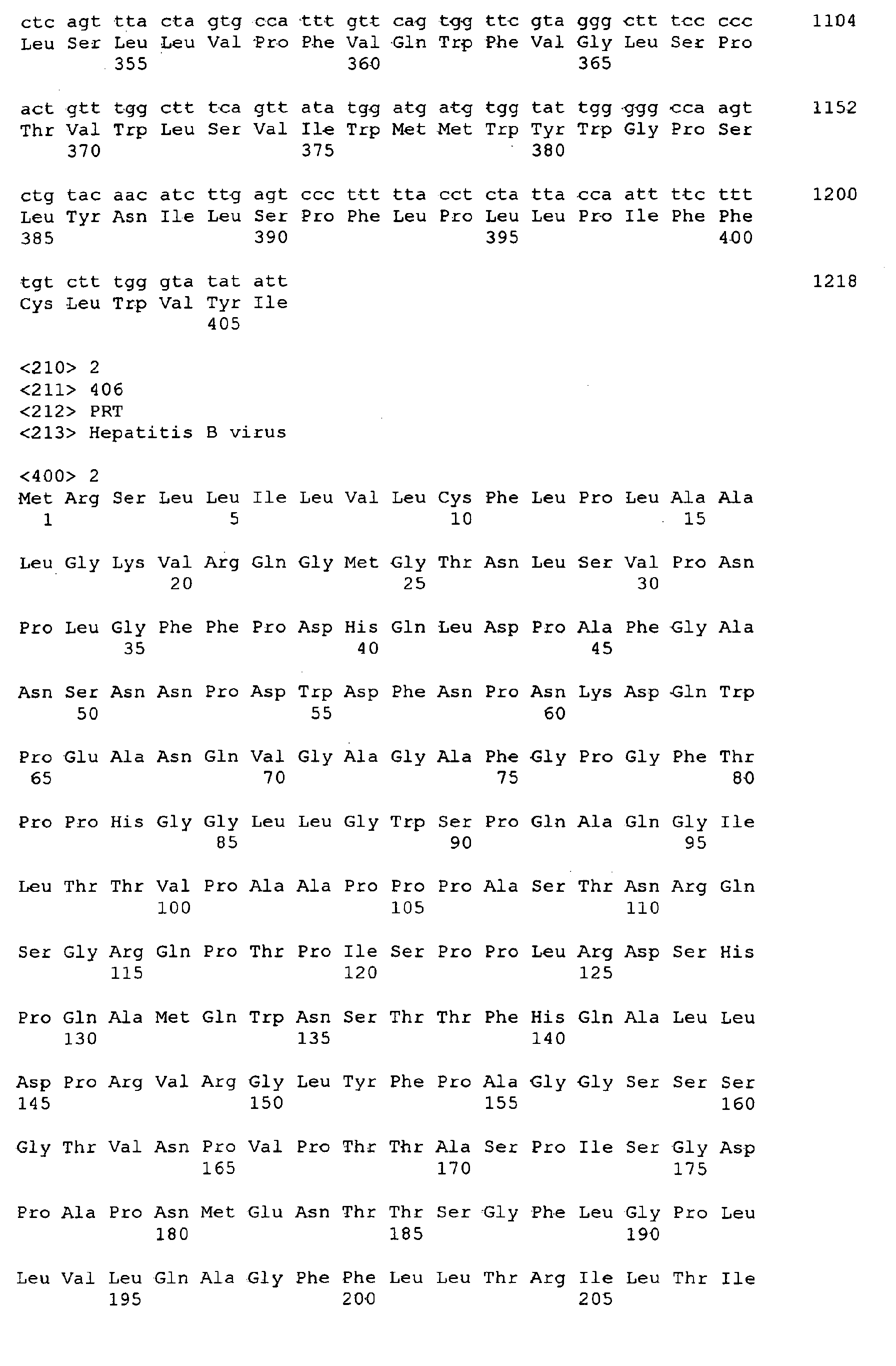

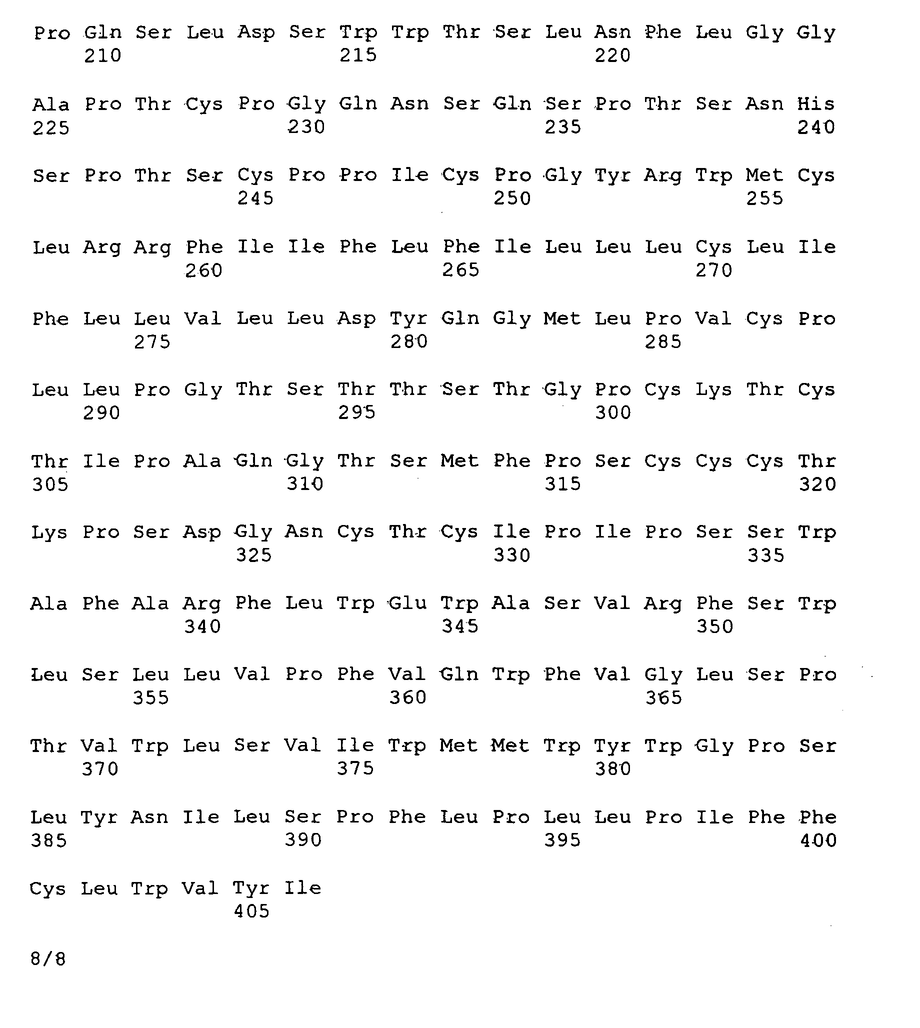

## Claims

1. A therapeutic product for therapy of hemophilia comprising
hollow nano particles formed of protein exhibiting a particle forming capability; and
genes for therapy of hemophilia embedded in said hollow nano particles.

2. A therapeutic product for therapy of hemophilia comprising
hollow nano particles formed by introducing bio-recognition molecules into protein particles obtained on expressing the protein in eucaryotic cells; and
genes for therapy of hemophilia embedded in said hollow nano particles.

3. The therapeutic product for therapy of hemophilia according to claim 2 wherein
said eucaryotic cells are yeast or recombinant yeast.

4. The therapeutic product for therapy of hemophilia according to claim 2 wherein
said eucaryotic cells are insect cells.

5. The therapeutic product for therapy of hemophilia according to claim 2 wherein
said eucaryotic cells are animal cells.

6. The therapeutic product for therapy of hemophilia according to any one of claims 1 to 5 wherein
said protein exhibiting the particle forming capability is hepatitis B virus surface antigen protein.

7. The therapeutic product for therapy of hemophilia according to any one of
claims 1 to 6 wherein
said gene for therapy of hemophilia is the clotting factor VIII or IX.

8. The therapeutic product for therapy of hemophilia according to any one of claims 1 to 7 wherein
the therapeutic product is administered to a human body by intravenous injection.

9. A method for therapy of hemophilia comprising administering the therapeutic product according to any one of claims 1 to 8.
